# EUROPEAN PATENT APPLICATION

(11) **EP 1 354 581 A1**
(43) Date of publication of application: **22.10.2003**
(21) Application number: 01271875.5
(22) Date of filing: 25.12.2001
(51) Int. Cl.: A61K 9/00, A61K 45/00, A61K 47/32, A61K 47/34, A61K 47/38, A61K 47/36, A61K 47/42, A61K 47/40

(54) **POROUS SUBSTANCE AND PROCESS FOR PRODUCING THE SAME**

(30) Priority: 26.12.2000 JP 2000395160
(71) Applicant: Takeda Chemical Industries, Ltd., Osaka-shi, Osaka 541-0045 (JP)
(72) Inventor: YOSHINARI, Tomohiro, Kobe-shi, Hyogo 651-1114 (JP); FUKUTA, Makoto, Nara-shi, Nara 631-0022 (JP); YOSHIOKA, Toshio, Toyonaka-shi, Osaka 560-0861 (JP)
(74) Representative: Rickard, Timothy Mark Adrian
(86) International application number: JP0111342
(87) International publication number: WO02051381

(57) **Abstract**

A physiologically active porous substance of the present invention obtained by treating a physiologically active solid substance with a carbon dioxide in a supercritical or subcritical state or a liquid carbon dioxide has a significantly improved dissolution rate and can easily be handled.

## Description

### Technical Field

The present invention relates to a porous substance having improved solubility and compression properties and to a method for producing the same.

### Background Art

In the pharmaceutical industry, it is known that the dissolution rate and the compression properties of a pharmaceutical compound can be improved by reducing the particle size by means of mechanical pulverization and control of the crystallization rate. The dissolution rate is represented by Noyes-Whitney formula: dC/dt=kS(Cs-C) wherein Cs is the saturated solubility of a solute, C is the concentration of the solute at time point "t", S is the surface area of a solid to be dissolved which is the solute, and k is the dissolution kinetic constant. To reduce the particle size means to increase the specific surface area of the solid to be dissolved mentioned above.

On the other hand, the improved compression properties are due to an increase of the binding sites resulting from reducing the particle size.

However, just reducing the particle size of a pharmaceutical has the disadvantages of dusting of the particles and a decrease in the flowability, as a result, the handling is difficult. Thus a process for reducing the particle size is not favorable for the subsequent formulation processes.

Accordingly, technology for improving the solubility and the compression properties of a pharmaceutical compound without posing the problems described above is desired.

### Disclosure of Invention

We made an effort to solve the problems described above and finally discovered that a physiologically active porous substance could be obtained by treating a physiologically active substance with carbon dioxide in a supercritical or subcritical state or liquid carbon dioxide. In addition, we found that the porous substance thus obtained had an unexpectedly improved dissolution rate and could be handled easily, and based on such findings we made a further effort and completed the present invention.

Thus, the present invention provides:
[1] a physiologically active porous substance obtained by treating a physiologically active solid substance with carbon dioxide in a supercritical or subcritical state or liquid carbon dioxide;
[2] a physiologically active porous substance whose weight-average particle size is about 1 µm or more and whose specific surface area is about 1.5 m²/g or more;
[3] a physiologically active porous substance according to the above-mentioned [2] wherein the weight-average particle size is about 10 µm or more and the specific surface area is about 1.5 m²/g or more;
[4] a physiologically active porous substance according to the above-mentioned [1] or [2] which is crystalline;
[5] a physiologically active porous substance according to the above-mentioned [1] or [2] wherein the physiologically active substance is a pharmaceutical compound;
[6] a physiologically active porous substance according to the above-mentioned [1] or [2] wherein the physiologically active solid substance is a sparingly water-soluble or water-insoluble substance whose solubility in water at 25°C is less than 10 mg/mL;
[7] a composition comprising a substance according to the above-mentioned [1] or [2];
[8] a composition according to the above-mentioned [7] comprising a surfactant or a high molecular compound;
[9] a composition according to the above-mentioned [8] wherein the high molecular compound is a high molecular polymer whose number-average molecular weight is about 3,000 to 30,000;
[10] a composition according to the above-mentioned [9] wherein the high molecular polymer is one or a copolymer or mixture of two or more selected from the group consisting of (1) poly-fatty acid ester, (2) poly-α-cyanoacrylate, (3) poly-hydroxybutyric acid, (4) polycarbonate selected from polyalkylene oxalate, poly-ortho-ester, poly-ortho-carbonate, polyethylene carbonate and polyethylene propylene carbonate, (5) polyamino acid, (6) polystyrene,
(7) polyacrylic acid, (8) polymethacrylic acid, (9) copolymer of acrylic acid and methacrylic acid, (10) silicon polymer, (11) dextran stearate, (12) ethyl cellulose, (13) acetyl cellulose, (14) nitrocellulose, (15) polyurethane, (16) maleic anhydride copolymer, (17) ethylene vinyl acetate copolymer, (18) polyvinyl acetate, (19) polyvinyl alcohol and (20) polyacrylamide;
[11] a composition according to the above-mentioned [9] wherein the high molecular polymer is polylactic acid, a lactic acid/glycolic acid copolymer, a 2-hydroxybutyric acid/glycolic acid copolymer or a mixture thereof;
[12] a composition according to the above-mentioned [8] wherein the high molecular compound is a hydrophilic polymer;
[13] a composition according to the above-mentioned [12] wherein the hydrophilic polymer is one or a mixture of two or more selected from the group consisting of (1) water-soluble polymer selected from hydroxyalkyl cellulose, cellulose derivative, polyalkenyl pyrrolidone, polyalkylene glycol and polyvinyl alcohol; (2) enteric polymer selected from hydroxypropylmethyl cellulose phthalate, hydroxypropylmethyl cellulose acetate succinate, carboxymethylethyl cellulose, cellulose acetate phthalate, methacrylic acid copolymer L and methacrylic acid copolymer S; (3) gastric soluble polymer selected from aminoalkyl methacrylate copolymer E and polyvinyl acetal diethylaminoacetate; (4) carboxymethyl cellulose; (5) Eudragit; (6) carboxyvinyl polymer; (7) polyvinyl alcohol; (8) gum arabic; (9) sodium alginate; (10) alginic acid propylene glycol ester; (11) agar; (12) gelatin and (13) chitosan;
[14] a composition according to the above-mentiond [7] comprising a readily water-soluble cyclodextrin derivative;
[15] a composition according to the above-mentioned [14] wherein the readily water-soluble cyclodextrin derivative is a compound represented by Formula: 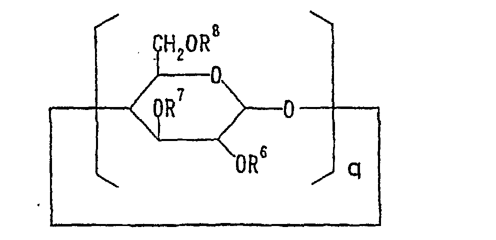 wherein q is an integer of 6 to 12, R⁶, R⁷ and R⁸ are same or different in individual repeating units and each is a dihydroxyalkyl group, sugar residue, hydroxyalkyl group or sulfoalkyl group;
[16] a composition according to the above-mentioned [15] wherein the dihydroxyalkyl group is a dihydroxy-C₁₋₆ alkyl group, the sugar residue is erythrosyl, threosyl, arabinosyl, ribosyl, glucosyl, galactosyl, glycero-gulco-heptosyl, maltosyl, lactosyl, maltotriosyl or dimaltosyl, the hydroxyalkyl group is a hydroxy-C₁₋₆ alkyl group and the sulfoalkyl group is a sulfo-C₁₋₆ alkyl group;
[17] a method for producing a physiologically active porous substance according to the above-mentioned [1] or [2] comprising treating a physiologically active solid substance with carbon dioxide in a supercritical or subcritical state or liquid carbon dioxide;
[18] a method according to the above-mentioned [17] wherein the supercritical or subcritical carbon dioxide or the liquid carbon dioxide is mixed with other solvents;
[19] a method according to the above-mentioned [17] comprising the steps of (1) placing a physiologically active solid substance in a pressure-resistant container, (2) keeping the temperature of said pressure-resistant container at a level allowing carbon dioxide to be in a supercritical or subcritical state, (3) filling carbon dioxide which may be optionally mixed with other solvents into said pressure-resistant container, (4) stopping filling the carbon dioxide at the time point when the pressure in said pressure-resistant container reaches a level allowing carbon dioxide to be in a supercritical or subcritical state, (5) depressurizing after completing the carbon dioxide treatment and then collecting the resultant physiologically active porous substance;
[20] a method according to any one of the above-mentioned [17] to [19] wherein the supercritical carbon dioxide is carbon dioxide in a state exceeding both the critical pressure of about 7.38 MPa and the critical temperature of about 304.1 K;
[21] a method according to any one of the above-mentioned [17] to [19] wherein the subcritical carbon dioxide is carbon dioxide in a state exceeding either the critical pressure of about 7.38 MPa or the critical temperature of about 304.1 K;
[22] a method according to the above-mentioned [17] comprising the steps of (1) placing a physiologically active substance in a pressure-resistant container, (2) keeping the temperature of said pressure-resistant container at the critical point of carbon dioxide or below, (3) filling carbon dioxide which may be optionally mixed with other solvents into said pressure-resistant container, (4) stopping filling the carbon dioxide under the condition where the pressure in said pressure-resistant container does not exceed the critical point of carbon dioxide, (5) depressurizing after completing the carbon dioxide treatment and then collecting the resultant physiologically active porous substance;
[23] a method according to the above-mentioned [18] or [19] wherein said other solvents are water, aromatic hydrocarbons, ethers, organochlorine organic solvents, alkylnitriles, nitroalkanes, amides, ketones, fatty acids, alcohols, sulfoxides or mixture solvents thereof;
[24] a method according to the above-mentioned [18] or [19] wherein said other solvents are water; aromatic hydrocarbons selected from benzene, toluene, ethyl acetate, cyclohexane and xylene; ethers selected from dimethyl ether, diethyl ether, dioxane, diethoxyethane, tetrahydrofuran and 1,2-dimethoxyethane; organochlorine organic solvents selected from dichloromethane, chloroform, carbon tetrachloride and 1,2-dichloroethane; alkylnitriles selected from acetonitrile and propionitrile; nitroalkanes selected from nitromethane and nitroethane; amides selected from N,N-dimethylformamide and N,N-dimethylacetoamide; acetone; fatty acids selected from acetic acid, acetic anhydride and oleic acid; alcohols selected from methanol, ethanol and propanol; dimethyl sulfoxide; or mixture solvents thereof;
[25] a method according to the above-mentioned [18] or [19] wherein said other solvent is ethanol or acetone; and
[26] a method according to the above-mentioned [18] or [19]
wherein the amount of said other solvents is about 1 to 50 % by volume based on the carbon dioxide which is in a supercritical, subcritical or liquid state.

### Brief Description of Drawings

Figure 1 shows a SEM photograph of the porous substance obtained in Example 1.
Figure 2 shows a magnified SEM photograph of the porous substance obtained in Example 1.
Figure 3 shows a SEM photograph of raw material of nifedipine.

### Best Mode for Carrying Out the Invention

Physiological active substances employed in the present invention can be selected from a wide range of substances including pharmaceutical compounds (including those for veterinary use), pesticidal compounds, fertilizers, cosmetics, perfumes, food materials, feeds, bactericides, fungicides, insect repellents, insecticides, antirusts and absorbents.

The characteristics of such physiologically active substances are not limited particularly. They are any of water-soluble, sparingly water-soluble or water-insoluble solid substances. Such physiologically active substances may also be crystalline.

The expression "sparingly water-soluble or water-insoluble" means that a physiologically active substance has a solubility in water at 25°C of less than 1000 ppm, preferably less than 10 ppm or that the solubility in water at 25°C is less than 10 mg/mL, preferably less than 0.1 mg/mL. The solubility can be measured by a standard method.

Water-soluble pharmaceutical compounds include those listed below.

### (1) Antibiotics

Tetracycline hydrochloride, ampicillin, piperacillin and the like.

### (2) Antipyretics, analgesics or antiphlogistics

Sodium salicylate, sulpirine, sodium indomethacin, morphine hydrochloride and the like.

### (3) Antitussives and expectorants

Ephedrin hydrochloride, noscapine hydrochloride, codeine phosphate, dihydrocodeine phosphate, isoproterenol hydrochloride and the like.

### (4) Sedatives

Chlorpromazine hydrochloride, atropine sulfate and the like.

### (5) Anti-ulcerative agents

Metachlopromide, histidine monohydrochloride and the like.

### (6) Anti-arrhythmic agents

Propranolol hydrochloride, alprenolol hydrochloride and the like.

### (7) Hypotenssive diuretics

Hexamethonium bromide, clonidine hydrochloride and the like.

### (8) Anti-coagulants

Heparin sodium, sodium citrate and the like.

Sparingly water-soluble or water-insoluble pharmaceutical compounds include those listed below.

### (1) Antipyretics, analgesics or antiphlogistics

Salicylic acid, sulpyrine, flufenamic acid, diclofenac, indomethacin, atropine, scopolamine, morphine, pethidine, levorphanol, ketoprofen, naproxen, ibuprofen, oxymorphone or a salt thereof and the like.

### (2) Ataractics

Diazepam, lorazepam, oxazepam and the like.

### (3) Anti-psychotics

Chlorpromazine, prochlorperazine, trifluoperazine and the like.

### (4) Antibacterial agents

Griseofulvin, Lankacidin [J. Antibiotics, 38, 877-885 (1985)], azole-based compounds [2-[(1R,2R)-2-(2,4-difluorophenyl)-2-hydroxy-1-methyl-3-(1H-1,2,4-triazol-1-yl)propyl]-4-[4-(2,2,3,3-tetrafluoropropoxy)]phenyl-3-(2H,4H)-1,2,4-triazolone, fluconazole, itraconazole and the like] and the like.

### (5) Antibiotics

Gentamycin, dibekacin, kanendomycin, lividomycin, tobramycin, amikacin, fradiomycin, sisomicin, tetracycline, oxytetracylcine, rolitetracycline, doxicycline, ampicillin, piperacillin, ticarcillin, cefalotin, cefaloridine, cefotiam, cefotiam-hexetil, cefsulodin, cefmenoxime, cefmetazole, cefazolin, cefotaxime, cefoperazone, ceftizoxime, moxalactam, thienamycin, sulfazecine, aztreonam or a salt thereof and the like.

### (6) Anti-tumor agents

6-O-(N-Chloroacetylcarbamoyl) fumagillol, bleomycin, methotrexate, actinomycin D, mitomycin C, daunorubicin, adriamycin, neocarzinostatin, cytosine arabinoside, fluorouracil, tetrahydrofuryl-5-fluorouracil, picibanil, lentinan, levamisole, bestatin, azimexon, glycyrrhizin and the like.

### (7) Anti-hyperlipidemic agents

Clofibrate, ethyl 2-chloro-3-[4-(2-methyl-2-phenylpropoxy)phenyl] propionate [Chem. Pharm. Bull., 38, 2792-2796 (1990)] and the like.

### (8) Antitussives and expectorants

Ephedrine, methylephedrine, noscapine, codeine, dihydrocodeine, alloclamide, clorphezianol, picoperidamine, cloperastine, protokylol, isoproterenol, salbutamol, tereputarine or a salt thereof and the like.

### (9) Muscle relaxants

Pridinol, tubocurarine, pancuronium and the like.

### (10) Antiepileptics

Phenytoin, ethosuximide, acetazolamide, chlordiazepoxide and the like.

### (11) Anti-ulcerative agents

Lansoprazole, metoclopramide and the like.

### (12) Antidepressants

Imipramine, clomipramine, noxiptiline, phenelzine and the like.

### (13) Anti-allergic agents

Diphenhydramine, chlorpheniramine, tripelennamine, metodiramine, clemizole, diphenylpyraline, methoxyphenamine and the like.

### (14) Cardiotonics

Trans-n-oxocamphor, terephylol, aminophylline, etilefrine and the like.

### (15) Anti-arrhythmic agents

Propranolol, alprenolol, bufetolol, oxprenolol and the like.

### (16) Vasodilators

Oxyfedrine, diltiazem, tolazoline, hexobendine, bamethan and the like.

### (17) Hypotensive diuretics

Hexamethonium bromide, pentolinium, mecamylamine, ecarazine, clonidine, diltiazem, nifedipin and the like.

### (18) Anti-diabetic agents

Glymidine, glipizide, phenformin, buformin, metformin and the like.

### (19) Anti-tuberculotic agents

Isoniazid, ethambutol, paraaminosalicylate and the like.

### (20) Narcotic antagonists

Levallorphan, nalorphine, naloxone or a salt thereof and the like.

### (21) Hormones

Steroid hormones, for example, dexamethasone, hexestrol, methimazole, betamethasone, triamcinolone, triamcinolone acetonide, fluocinolon acetonide, prednisolone, hydrocortisone, estriol and the like.

### (22) Fat-soluble vitamins

[1] Vitamin K: Vitamin K₁, K₂, K₃ and K₄.
[2] Folic acid (vitamin M) and the like.

### (23) Vitamin derivatives

Various vitamin derivatives, for example, vitamin D₃ derivatives such as 5,6-trans-cholecalciferol, 2,5-hydroxycholecalciferol, 1-α-hydroxycholecalciferol, vitamin D₂ derivatives such as 5,6-trans-ergocalciferol and the like.

### (24) Others

Hydroxycam, diaserine, megestrol acetate, nicergoline, prostaglandins and the like.

In addition, therapeutic agents for ischemic disease, immune disease, Alzheimer's disease, osteoporosis, neovascularization, retinopathy, retinal vein occlusion, senile discoid macular degeneration, cerebrovascular spasm, cerebral thrombosis, cerebral infarction, cerebral occlusion, intracerebral hemorrhage, subarachnoid hemorrhage, hypertensive encephalopathy, transient cerebral ischemia attack, multi-infarct dementia, arterial sclerosis, Huntington's disease, cerebral tissue impairment, optic neuropathy, glaucoma, ocular hypertension, retinal detachment, arthritis, rheumatism, sepsis, septic shock, asthma, pollakiuria, urinary incontinence, atopic dermatitis, allergic rhinitis and the like are also employed.

Sparingly water-soluble or sparingly soluble solid pesticidal compounds include those listed below.

### (1) Insecticides

### (a) Carbamate

MIPC; isoprocarb, BPMC; fenobucarb, MPMC; xylylcarb, XMC, NAC; carbaryl, bendiocarb, carbofuran and the like.

### (b) Synthetic pyrethroids

Cypermethrin, fenpropathrin, ethofenprox, resmethrin and the like.

### (c) Organophosphorus

EPN, cyanofenphos, PAP; phenthoate, CVMP; tetrachlorvinphos, monocrotophos, phosalone, chlorpyrifos-methyl, chlorpyrifos, pyridaphenthion, quinalphos, DMTP; methidathion, dioxabenzofos and the like.

### (d) Organochlorine

Endosulfan and the like.

### (e) Others

Bensultap, buprofezin, flufenoxuron, diflubenzuron, chlorfluazuron, imidacloprid and the like.

### (2) Bactericides

### (a) N-Heterocyclic ergosterol inhibitors

Triflumizole, triforine and the like.

### (b) Carboxyamide

Mepronil, flutoluanil, pencycuron, oxycarboxin and the like.

### (c) Dicarboxyimide

Iprodione, vinclozolin, procymidone and the like.

### (d) Benzoimidazole

Benomyl and the like.

### (e) Polyhaloalkylthio

Captan and the like.

### (f) Organochlorine

Fthalide, TPN; chlorothalonil and the like.

### (g) Sulfur

Zineb, maneb and the like.

### (h) Others

Diclomezin, tricyclazole, isoprothiolane, probenazole, anilazine, oxolinic acid, ferimzone and the like.

### (3) Herbicides

### (a) Sulfonylurea

Imazosulfuron, bensulfuron-methyl and the like.

### (b) Triazine

Simetryn, dimethametryn and the like.

### (c) Urea

Dymron and the like.

### (d) Acid amide

Propanil, mefenacet and the like.

### (e) Carbamate

Swep and the like.

### (f) Diazole

Oxadiazon, pyrazolate and the like.

### (g) Dinitroaniline

Trifluralin and the like.

### (h) Others

Dithiopyr and the like.

A physiologically active porous substance according to the present invention (hereinafter abbreviated as the inventive porous substance) can be produced, for example, by treating the physiologically active substance described above with carbon dioxide in a supercritical or subcritical state (including a critical state) or liquid carbon dioxide.

A process for such a treatment may be any process capable of avoiding complete dissolution of the physiologically active substance in carbon dioxide in a supercritical or subcritical state or liquid carbon dioxide, which may for example be a dispersing (preferably, just dispersing rather than dissolving completely), wetting, infiltrating, contacting or mixing process, with a dispersing (preferably, just dispersing rather than dissolving completely) process being preferred especially.

A supercritical state means a state in which both pressure and temperature exceed the respective critical points.

A subcritical state means a state in which either pressure or temperature exceeds the critical point.

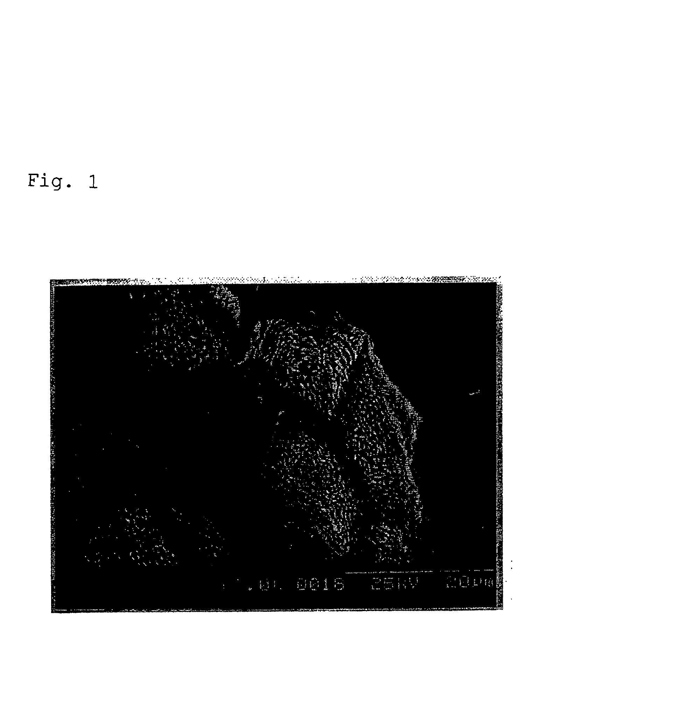

A critical point is defined, for example, by J.W.Tom and P.G.Debenedetti in Fig.1 in "Particle Formation with Supercritical Fluids-A Review", J. Aerosol Sci., 22(5), p.555-584 (1991).

A state in which neither pressure nor temperature exceeds the respective critical points is referred to as liquid.

Typically, carbon dioxide in a supercritical state is in a state exceeding both the critical pressure of about 7.38 megapascal (MPa) and the critical temperature of about 304.1 kelvin (K).

When a physiologically active substance is treated with carbon dioxide in a supercritical or subcritical state or liquid carbon dioxide, the ratio of the carbon dioxide to the physiologically active substance may vary depending on the size and type of a container employed. Usually, about 0.5 g to 20 kg, preferably about 10 g to 2 kg of a physiologically active substance is treated with about 50 ml to 2000 L of carbon dioxide in a supercritical or subcritical state or liquid carbon dioxide.

The treatment period is usually about 1 minute to 24 hours, preferably about 0.05 to 12 hours, more preferably about 5 to 120 minutes.

The treatment for example by dispersing is preferably usually conducted in a pressure-resistant container. For example, a supercritical fluid extraction system SCF-get (NIPPON BUNKO) (said system consists of a supercritical CO₂ supply pump SCF-get, a fully automatic pressure adjusting valve SGF-Bpq and a thermostat chamber CO-1560) may be employed.

The treatment temperature is usually about 40°C to about 100°C, while it may vary depending on the type of the starting physiologically active substance. For example, when the starting physiologically active substance is nifedipine, then the temperature is about 30°C to about 60°C.

The treatment pressure is usually about 3 megapascal (MPa) to about 50 MPa, while it may vary depending on the type of the starting physiologically active substance. For example, when the starting physiologically active substance is nifedipine, then the pressure is about 15 MPa to about 20 MPa.

For the treatment, other solvents in addition to carbon dioxide may be employed, for example, as a mixture thereof.

Such other solvents include water; aromatic hydrocarbons such as benzene, toluene, ethyl acetate, cyclohexane and xylene; ethers such as dimethyl ether, diethyl ether, dioxane, diethoxyethane, tetrahydrofuran and 1,2-dimethoxyethane; organochlorine organic solvents such as dichloromethane, chloroform, carbon tetrachloride and 1,2-dichloroethane; alkylnitriles such as acetonitrile and propionitrile; nitroalkanes such as nitromethane and nitroethane; amides such as N,N-dimethylformamide and N,N-dimethylacetoamide; ketones such as acetone; fatty acids such as acetic acid, acetic anhydride and oleic acid; alcohols such as methanol, ethanol and propanol; sulfoxides such as dimethyl sulfoxide; and mixtures solvents thereof, with ethanol or acetone being preferred.

The amount of such other solvents is about 0.1 to 99.9% by volume, preferably about 1 to 50 % by volume based on the carbon dioxide in a supercritical, subcritical or liquid state.

More specifically, the inventive porous substance can be produced in accordance with the procedure (1) or (2) described below.

### (1) Procedure 1

[1] Place a physiologically active substance in a pressure-resistant container,
[2] Keep the temperature of said pressure-resistant container at a level allowing carbon dioxide to be in a supercritical or subcritical state,
[3] Fill carbon dioxide (if necessary in a mixture with other solvents) into said pressure-resistant container,
[4] Stop filling the carbon dioxide at the time point when the pressure in said pressure-resistant container reaches a level allowing carbon dioxide to be in a supercritical or subcritical state,
[5] Depressurize after completing the carbon dioxide treatment and then collect the resultant physiologically active porous substance;

Preferably,
[1] Place a physiologically active substance in a pressure-resistant container,
[2] Keep the temperature of said pressure-resistant container at a level allowing carbon dioxide to be in a supercritical or subcritical state,
[3] Fill carbon dioxide (if necessary in a mixture with other solvents) from a cylinder connected into said pressure-resistant container,
[4] Stop filling the carbon dioxide at the time point when the pressure in said pressure-resistant container reaches a level allowing carbon dioxide to be in a supercritical or subcritical state,
[5] Depressurize after allowing to stand for about 1 minute to 24 hours (preferably about 5 to 120 minutes) and then collect the resultant physiologically active porous substance.

### (2) Procedure 2

[1] Place a physiologically active substance in a pressure-resistant container,
[2] Keep the temperature of said pressure-resistant container at the critical point of carbon dioxide or below,
[3] Fill carbon dioxide (if necessary in a mixture with other solvents) into said pressure-resistant container,
[4] Stop filling the carbon dioxide under the condition where the pressure in said pressure-resistant container does not exceed the critical point of carbon dioxide,
[5] Depressurize after completing the carbon dioxide treatment and then collect the resultant physiologically active porous substance;

Preferably,
[1] Place a physiologically active substance in a pressure-resistant container,
[2] Keep the temperature of said pressure-resistant container at the critical point of carbon dioxide or below,
[3] Fill carbon dioxide (if necessary in a mixture with other solvents) from a cylinder connected into said pressure-resistant container,
[4] Stop filling the carbon dioxide under the condition where the pressure in said pressure-resistant container does not exceed the critical point of carbon dioxide,
[5] Depressurize after allowing to stand for about 1 minute to 24 hours (preferably about 5 to 120 minutes) and then collect the resultant physiologically active porous substance.

By allowing to stand for about 1 minute to 24 hours in Step [5], the physiologically active solid substance is infiltrated or wetted with the carbon dioxide.

In the production method according to the present invention, a part of the starting physiologically active solid substance, which is not dissolved in the carbon dioxide, becomes porous.

The inventive porous substance thus obtained usually has a weight-average particle size of 1 µm or more, preferably about 1 µm to about 2000 µm. More preferably, the weight-average particle size is 10 µm or more, particularly about 10 µm to about 500 µm. The weight-average particle size can be measured by a laser diffraction method.

The specific surface area of the inventive porous substance is usually about 1.5 m²/g or more, preferably about 1.5 m²/g to about 100 m²/g, more preferably about 1.5 m²/g to about 50 m²/g, while the upper limit is not set particularly. The specific surface area can be measured by a BET method.

The inventive porous substance is preferably crystalline.

The inventive porous substance has an increased specific surface area as compared with the starting physiologically active substance, which leads to an improved dissolution rate. In addition, it has a sufficient flowability, can be handled readily and allows formulation to be accomplished easily.

The term "improved water solubility" means, for example, an increased dissolution rate in water. Typically, it means that a dissolution rate in water at 25°C increases by about 2 times, preferably about 5 times, more preferably about 10 times, especially 100 times or more.

A composition containing the inventive porous substance (hereinafter referred to as the inventive composition) can be used as appropriate depending on the type of a physiologically active substance contained as an active ingredient.

The inventive composition may contain appropriate additives. Preferred additives include surfactants or high molecular compounds capable of modifying the surface of the porous substance, which may be employed alone or in a combination of two or more of them.

Surfactants for use include nonionic surfactants, anionic surfactants, cationic surfactants, amphoteric surfactants and naturally occurring surfactants.

Nonionic surfactants include higher alcohol ethylene oxide adducts, alkylphenol ethylene oxide adducts, fatty acid ethylene oxide adducts, polyhydric alcohol fatty acid ester ethylene oxide adducts, higher alkylamine ethylene oxide adducts, fatty acid amide ethylene oxide adducts, fat ethylene oxide adducts, glycerin fatty acid esters, pentaerythritol fatty acid esters, polyhydric alcohol alkyl ethers, and fatty acid amides of alkanolamines.

Among the nonionic surfactants listed above, those employed preferably are fatty acid esters of sorbitol and sorbitan, polyoxyethylene sorbitan fatty acid esters, polyethylene glycol fatty acid esters, sucrose fatty acid esters, polyethoxylated castor oil, polyethoxylated hydrogenated castor oil, polyoxyethylene polypropylene glycol copolymers, glycerin fatty acid esters, polyglycerin fatty acid esters and the like. The sorbitan fatty acid ester is preferably sorbitan monostearate (trade name: SS-10, NIKKO CHEMICALS), sorbitan sesquioleate (trade name: SO-15, NIKKO CHEMICALS), sorbitan trioleate (trade name: SO-30, NIKKO CHEMICALS) or the like. The polyoxyethylene sorbitan fatty acid ester is preferably Polysorbate 20 (trade name: TL-10, NIKKO CHEMICALS), 40 (trade name: TP-10, NIKKO CHEMICALS), 60 (trade name: TS-10, NIKKO CHEMICALS), 80 (trade name: TO-10, NIKKO CHEMICALS) or the like. The polyethylene glycol fatty acid ester is preferably polyethylene glycol monolaurate (10E.O.) (trade name: MYL-10, NIKKO CHEMICALS) or the like. The sucrose fatty acid ester is preferably sucrose palmitate (for example, trade name: S-1670, MITSUBISHI KAGAKU FOODS), sucrose stearate (for example, trade name: P-1670, MITSUBISHI KAGAKU FOODS) or the like. The polyethoxylated castor oil is preferably polyoxyethylene glycerol triricinoleate 35 (Polyoxy 35 Castor Oil, trade name: Cremophor EL or EL-P, BASF Japan) or the like. The polyethoxylated hydrogenated castor oil is preferably Polyoxyethylene Hydrogenated Castor Oil 50, Polyoxyethylene Hydrogenated Castor Oil 60 or the like. The polyoxyethylene polyoxypropylene glycol copolymer is preferably polyoxyethylene (160) polyoxypropylene (30) glycol (trade name: Adeka Pluronic F-68, ASAHI DENKA KOGYO) or the like. The glycerin fatty acid ester is preferably glyceryl monostearate (MGS series, NIKKO CHEMICALS) or the like. The polyglycerin fatty acid ester is preferably tetraglycerin monostearic acid (MS-310, SAKAMOTO YAKUHIN KOGYO), decaglycerin monolauric acid (Decaglyn 1-L, NIKKO CHEMICALS) or the like.

Anionic surfactants include sulfates (e.g., higher alcohol sulfuric acid ester salts, higher alkyl ether sulfuric acid ester salts, sulfated oils, sulfated fatty acid esters, sulfated fatty acids, sulfated olefins), sulfonates (e.g., sodium alkylbenzene sulfonates, oil-soluble alkylbenzene sulfonates, α-olefin sulfonates, Igepon type T, aerosol type OT), phosphates (e.g., phosphoric acid ester salts of higher alcohol ethylene oxide adducts), and dithiophosphoric acid ester salts.

Among the anionic surfactants listed above, those employed preferably are bile acid salts such as sodium glycocholate or sodium deoxycholate, fatty acids and their salts such as stearic acid or sodium caprate, sodium lauryl sulfate and the like.

Cationic surfactants include cationic surfactants of an amine salt type (e.g., cationic surfactants of an amine salt type produced from higher alkylamine, cationic surfactants of an amine salt type produced from lower alkylamine) and cationic surfactants of a quaternary ammonium salt type (e.g., cationic surfactants of a quaternary ammonium salt type produced from higher alkylamine, cationic surfactants of a quaternary ammonium salt type produced from lower alkylamine).

Amphoteric surfactants include amphoteric surfactants of an amino acid type and amphoteric surfactants of a betaine type.

Naturally occurring surfactants include lecithin such as purified egg yolk lecithin (trade name: PL-100H, QP) or hydrogenated soybean lecithin (trade name: Lecinol S-10, NIKKO CHEMICALS) .

Among the surfactants listed above, those employed preferably are sodium deoxycholate, sodium lauryl sulfate, Polysorbate 80, polyoxyethylene (160) polyoxypropylene (30), polyoxyethylene glycerol triricinoleate 35, lecithins, polyethoxylated hydrogenated castor oils, sucrose fatty acid esters and polyglycerin fatty acid esters, with sodium deoxycholate being more preferred.

Any of these surfactants may be employed alone or in a combination of two or more.

High molecular compounds for use include high molecular polymers and hydrophilic polymers.

The number-average molecular weight of a high molecular polymer is preferably about 3,000 to 30,000, more preferably about 5,000 to 25,000, especially about 5,000 to 20,000. When used herein, the terms "weight-average molecular weight" and "dispersion degree" mean those measured by gel permeation chromatography (GPC) using polystyrene as a reference standard. The measurement uses a GPC column KF804 x 2 (SHOWA DENKO) together with chloroform as a mobile phase.

Examples of such a high molecular polymer, for example, of an in vivo degradable type are:
[1] poly-fatty acid esters [e.g. homopolymers of fatty acids (e.g., polylactic acid, polyglycolic acid, polycitric acid, polymalic acid and the like), copolymers of two or more fatty acids (e.g., a lactic acid/glycolic acid copolymer, a 2-hydroxybutyric acid/glycolic acid copolymer and the like), mixtures thereof (e.g., a mixture of polylactic acid and 2-hydroxybutyric acid/glycolic acid copolymer), wherein said fatty acids include α-hydroxyfatty acids (e.g., glycolic acid, lactic acid, 2-hydroxybutyric acid, 2-hydroxyvaleric acid, 2-hydroxy-3-methylbutyric acid, 2-hydroxycaproic acid, 2-hydroxyisocaproic acid, 2-hydroxycapric acid and the like), cyclic dimers of α-hydroxyfatty acids (e.g., glycoside, lactide and the like), hydroxydicarboxylic acids (e.g., malic acid and the like), hydroxytricarboxylic acids (e.g., citric acid and the like);
[2] Poly-α-cyanoacrylates;
[2] Polyhydroxybutyric acids;
[3] Polyalkylene oxalates (e.g., polytrimethylene oxalates, polytetramethylene oxalates and the like);
[4] Polyorthoesters, polyorthocarbonates or other polycarbonates (e.g., polyethylene carbonates, polyethylene propylene carbonates and the like);
[5] Polyamino acids (e.g., poly-γ-benzyl-L-glutamic acids, poly-L-alanines, poly-γ-methyl-L-glutamic acids and the like); and the like.

In addition, other biocompatible high molecular polymers such as polystyrenes, polyacrylic acids, polymethacrylic acid, copolymers of acrylic acid and methacrylic acid, silicon polymers, dextran stearate, ethyl cellulose, acetyl cellulose, nitrocellulose, polyurethanes, maleic anhydride copolymers, ethylene vinyl acetate copolymers, polyvinyl acetates, polyvinyl alcohols, polyacrylamides and the like are included. Any of these polymers may be employed alone, as a copolymer or just a mixture of two or more, or may be employed as a salt thereof.

Among the high molecular polymers listed above, those employed preferably are poly-fatty acid esters and poly-α-cyanoacrylates. Those employed more preferably are poly-fatty acid esters.

Among poly-fatty acid esters, those employed preferably are homopolymers of α-hydroxyfatty acid or a cyclic dimer of α-hydroxyfatty acid, copolymers of two or more α-hydroxyfatty acids or cyclic dimers of α-hydroxyfatty acids, as well as the mixtures thereof. Those employed more preferably are homopolymers of α-hydroxyfatty acids, copolymers of two or more α-hydroxyfatty acids or mixtures thereof. Those preferred especially are polylactic acids, lactic acid/glycolic acid copolymers, 2-hydroxybutyric acid/glycolic acid copolymers as well as the mixtures thereof.

These α-hydroxycarboxylic acids may be in the D-form, L-form or D,L-form, if present, with the D,L-form being employed preferably.

When a lactic acid/glycolic acid copolymer is employed as the high molecular polymer described above, its ratio is preferably about 100/0 to 50/50. When a butyric acid-glycolic acid copolymer is employed, its ratio is preferably about 100/0 to 25/75.

The weight-average molecular weight of the lactic acid/glycolic acid copolymer is preferably about 5,000 to about 30,000, more preferably about 5,000 to 20,000.

When a mixture of polylactic acid (A) and a glycolic acid/2-hydroxybutyric acid copolymer (B) is employed as the high molecular polymer described above, the ratio represented by (A)/(B) is within the range from about 10/90 to about 90/10 (weight ratio), preferably within the range from about 25/75 to about 75/25.

The weight-average molecular weight of the polylactic acid is preferably about 5,000 to about 30,000, more preferably about 6,000 to about 20,000.

The glycolic acid/2-hydroxybutyric acid copolymer is preferably composed of about 40 to 70 moles of glycolic acid, with the reminder being 2-hydroxybutyric acid. The weight-average molecular weight of the glycolic acid/2-hydroxybutyric acid copolymer is preferably about 5,000 to about 25,000, more preferably about 5,000 to about 20,000.

Hydrophilic polymers include water-soluble polymers, enteric polymers and gastric soluble polymers.

Water-soluble polymers include cellulose derivatives including hydroxyalkyl cellulose such as hydroxypropyl cellulose and hydroxypropylmethyl cellulose and alkyl cellulose such as methyl cellulose, polyalkenyl pyrrolidone such as polyvinyl pyrrolidone, polyalkylene glycol such as polyethylene glycol and polyvinyl alcohol.

Enteric polymers include hydroxypropylmethyl cellulose phthalate, hydroxypropylmethyl cellulose acetate succinate, carboxymethylethyl cellulose, cellulose acetate phthalate, methacrylic acid copolymer L and methacrylic acid copolymer S.

Gastric soluble polymers include aminoalkyl methacrylate copolymer E and polyvinyl acetal diethyl aminoacetate.

In addition, carboxymethyl cellulose, Eudragit, a carboxyvinyl polymer, polyvinyl alcohol, gum arabic, sodium alginate, alginic acid propylene glycol ester, agar, gelatin, chitosan and the like can be also employed. Any of these hydrophilic polymers may be employed alone or in a combination of two or more.

The inventive composition may also contain a readily water-soluble cyclodextrin derivative for the purpose of further improving the solubility of the porous substance.

Such a readily water-soluble cyclodextrin derivative may be commercially available, or can be produced in accordance with a method known per se.

Such a readily water-soluble cyclodextrin derivative is preferably a compound in which a part or all of the hydrogen atoms of the hydroxyl groups in the 2, 3 and 6-positions on a glucose in a cyclic oligosaccharide consisting of 6 to 12 glucose units are substituted by other functional groups (for example, dihydroxyalkyl groups, sugar residues, hydroxyalkyl groups, sulfoalkyl groups).

Such a readily water-soluble cyclodextrin derivative has a solubility in water of about 100 mg/ml or more, preferably about 130 mg/ml or more.

A preferred example of such a readily water-soluble cyclodextrin derivative is a compound represented by Formula (I): 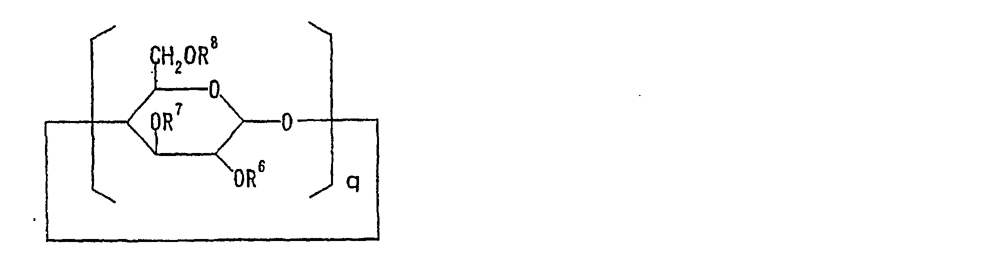 wherein q is an integer of 6 to 12, R⁶, R⁷ and R⁸ are same or different in individual repeating units and each is a dihydroxyalkyl group, sugar residue, hydroxyalkyl group or sulfoalkyl group. Those exemplified typically are α-CyD(q=6), β-CyD(q=7), γ-CyD(q=8) and δ-CyD(q=9) whose hydroxyl groups are ether-derivatized. Among these, β-CyD whose hydroxyl groups are ether-derivatized is preferred.

The dihydroalkyl group represented by R⁶ to R⁸ may for example be a dihydroxy-C₁₋₆ alkyl group (e.g., dihydroxymethyl, 2,2-dihydroxyethyl, 2,2-dihydroxypropyl, 2,2-dihydroxypentyl, 2,2-dihydroxyhexyl), preferably a dihydroxy-C₁₋₄ alkyl group (e.g., dihydroxymethyl, 2,2-dihydroxyethyl, 2,2-dihydroxypropyl).

The sugar residue represented by R⁶ to R⁸ may for example be a C₃₋₂₄ sugar residue (erythrosyl, threosyl, arabinosyl, ribosyl, glucosyl, galactosyl, glycero-gulco-heptosyl, maltosyl, lactosyl, maltotriosyl or dimaltosyl), preferably a C₆₋₂₄ sugar residue (e.g., glucosyl, galactosyl, glycero-gulco-heptosyl, maltosyl, lactosyl, maltotriosyl and dimaltosyl), more preferably a C₆₋₁₂ sugar residue (e.g., glucosyl, galactosyl, glycero-gulco-heptosyl, maltosyl, lactosyl).

The hydroxyalkyl group represented by R⁶ to R⁸ may for example be a hydroxy-C₁₋₆ alkyl group (e.g., hydroxymethyl, 2-hydroxyethyl, 2-hydroxypropyl, 2-hydroxypentyl, 2-hydroxyhexyl), preferably a hydroxy-C₁₋₄ alkyl group (e.g., hydroxymethyl, 2-hydroxyethyl, 2-hydroxypropyl), especially a 2-hydroxypropyl group.

The sulfoalkyl group represented by R⁶ to R⁸ may for example be a sulfo-C₁₋₆ alkyl group (e.g., sulfomethyl, sulfoethyl, sulfopropyl, sulfopentyl, sulfohexyl), preferably a sulfo-C₁₋₄ alkyl group (e.g., sulfomethyl, sulfoethyl, sulfopropyl), especially a sulfobutyl group.

A further preferred example of such a readily water-soluble cyclodextrin derivative is a compound represented by Formula (II) wherein at least one of R⁶ to R⁸ is a sugar residue, hydroxyalkyl group or sulfoalkyl group.

A compound (II) in which at least one of R⁶ to R⁸ is a sugar residue includes glucosyl-α,β,γ,δ-CyD, maltosyl-α,β,γ,δ-CyD, maltotriosyl-α,β,γ,δ-CyD and dimaltosyl-α,β,γ,δ-CyD. Among these, maltosyl-α,β,γ,δ-CyD and glucosyl-α,β,γ,δ-CyD are preferred. Maltosyl-β-CyD (hereinafter abbreviated as G2-β-CyD) and glucosyl-β-CyD are especially preferred.

A compound (II) in which at least one of R⁶ to R⁸ is a hydroxyalkyl group includes hydroxypropyl-α,β,γ, δ-CyD. Among these, hydroxypropyl-β-CyD is preferred especially.

A compound (II) in which at least one of R⁶ to R⁸ is a sulfoalkyl group includes sulfobutyl-α,β,γ,δ-CyD. Among these, sulfobutyl-β-CyD is preferred especially.

In addition, branched cyclodextrin-carboxylic acid can be also used as the readily water-soluble cyclodextrin derivative. The branched cyclodextrin-carboxylic acid includes not only its free carboxylic acid but also its alkaline metal salts (e.g., lithium, sodium, potassium) and alkaline earth metal salts (e.g., calcium, magnesium). Any of these branched cyclodextrin-carboxylic acids may be employed alone or in a combination of two or more, or also in a mixture of free carboxylic acid with its salt.

Such branched cyclodextrin-carboxylic acid is cyclodextrin in which an organic group containing at least one carboxyl group is in the 6-O position on at least one glucose unit of said cyclodextrin ring.

The cyclodextrin ring of said branched cyclodextrin-carboxylic acid has, for example, 6, 7 or 8 glucose units. Preferably said cyclodextrin ring has 7 glucose units. Such cyclodextrin includes α-cyclodextrin, β-cyclodextrin and γ-cyclodextrin.

In a preferred case, the organic group having at least one carboxyl group has 1 to 3 glucose units, and at least one hydroxylmethyl group in the glucose unit in said organic group is oxidized into a carboxylic group.

Examples of such branched cyclodextrin-carboxylic acid are 6-O-cyclomaltohexaosyl-(6 → 1)-α-D-glucosyl-(4 → 1)-O-α-D-glucuronic acid (cyclomaltohexaosyl-(6 → 1)-α-D-glucopyranosyl-(4 → 1)-O-α-D-glucopyranoside uronic acid) (hereinafter sometimes abbreviated as α-CyD-G₂-COOH; the following compounds are also abbreviated similarly in brackets), 6-O-cyclomaltoheptaosyl-(6 → 1)-α-D-glucosyl-(4 → 1)-O-α-D-glucuronic acid (cyclomaltoheptaosyl-(6 → 1)-O-α-D-glucopyranosyl-(4 → 1)-O-a-D-glucopyranoside uronic acid) (β-CyD-G₂-COOH), 6-O-cyclomaltooctaosyl-(6 → 1)-α-D-glucosyl- (4 → 1)-O-α-D-glucuronic acid (cyclomaltooctaosyl-(6 → 1)-O-α-D-glucopyranosyl-(4 → 1)-O-α-D-glucopyranoside uronic acid) (γ-CyD-G₂-COOH), 6-0-cyclomaltohexaosyl-(6 → 1)-α-D-glucuronic acid (cyclomaltohexaosyl- (6 → 1)-O-a-D-glucopyranoside uronic acid) (α-CyD-G₁-COOH), 6-O-cyclomaltoheptaosyl-(6 → 1)-α-D-glucuronic acid (cyclomaltoheptaosyl-(6 → 1)-O-α-D-glucopyranoside uronic acid) (β-CyD-G₁-COOH), 6-O-cyclomaltooctaosyl-(6 → 1)-α-D-glucuronic acid (cyclomaltooctaosyl-(6 → 1)-O-α-D-glucopyranoside uronic acid) (γ-CyD-G₁-COOH), 2-O-(6-cyclomaltohexaosyl)-acetic acid (α-CyD-CH₂COOH), 2-O-(6-cyclomaltoheptaosyl)-acetic acid (β-CyD-CH₂COOH), 2-O-(6-cyclomaltooctaosyl)-acetic acid (γ-CyD-CH₂COOH), 3-O-(6-cyclomaltoheptaosyl)-propionic acid (β-CyD-CH₂ CH₂COOH), 2-hydroxy-3-O-(6-cyclomaltoheptaosyl) -propionic acid (3-O-(6-cyclomaltoheptaosyl) -2-hydroxy-propionic acid) (β-CyD-CH₂CH(OH)-COOH), 7^{A}, 7^{C}-di-O-[α-D-glucuronyl-(1 → 4) -O-α-D-glucosyl]-(1 → 6)-maltoheptaose (β-CyD-(G₂COOH)₂), 6-O-cyclomaltoheptaosyl-O-α-D-maltosyl-(4 → 1)-O-α-D-glucuronic acid (cyclomaltoheptaosyl-(6 → 1)-O-α-D-glucopyranosyl-(4 → 1)-O-α-D-glucopyranosyl-(4 → 1)-O-α-D-glucopyranoside uronic acid) (β-CyD-G₃-COOH) as well as their salts [e.g., sodium salt of β-CyD-G₂-COOH (sodium cyclomaltoheptaosyl-(6 → 1)-O-α-D-glucopyranosyl-(4 → 1)-O-α-D-glucopyranoside uronate (similarly abbreviated as β-CyD-G₂-COONa))]. Among those listed above, β-CyD-G₂-COONa is preferred.

More particularly, 6-O-cyclomaltohexaosyl- (6 → 1)-α-D-glucosyl-(4 → 1)-O-α-D-glucuronic acid (α-CyD-G₂-COOH), 6-O-cyclomaltoheptaosyl-(6 → 1)-α-D-glucosyl-(4 → 1)-O-α-D-glucuronic acid (β-CyD-G₂-COOH) and 6-O-cyclomaltooctaosyl-α-D-glucosyl- (4 → 1)-O-α-D-glucuronic acid (γ-CyD-G₂-COOH) are branched cyclodextrin-carboxylic acids containing α-cyclodextrin (6 glucose units), β-cyclodextrin (7 glucose units) and γ-cyclodextrin (8 glucose units) respectively, wherein maltose is linked to one of the glucose units of the cyclodextrin ring via an α-(1 → 6) bond and the hydroxymethyl group in the 6-position on the terminal glucose of said maltose is oxidized into a carboxylic group, whereby forming glucuronic acid.

On the other hand, 6-O-cyclomaltohexaosyl- (6 → 1)-α-D-glucuronic acid (α-CyD-G₁-COOH), 6-O-cyclomaltoheptaosyl-(6 → 1)-α-D-glucuronic acid (β-CyD-G₁-COOH) and 6-O-cyclomaltooctaosyl-(6 → 1)-α-D-glucuronic acid (γ-CyD-G₁-COOH) are branched cyclodextrin-carboxylic acids wherein glucose is linked to one glucose unit of the cyclodextrin ring via an α-(1 → 6) bond and the hydroxymethyl group in the 6-position on said branched glucose is oxidized into a carboxylic group, whereby forming glucuronic acid.

Moreover, 2-O-(6-cyclomaltohexaosyl)-acetic acid (α-CyD-CH₂COOH), 2-O-(6-cyclomaltoheptaosyl)-acetic acid (β-CyD-CH₂COOH) and 2-O-(6-cyclomaltooctaosyl)-acetic acid (γ-CyD-CH₂COOH) are the branched cyclodextrin-carboxylic acids, wherein a carboxymethyl group is linked to one glucose unit of the cyclodextrin ring to form a branch.

These branched cyclodextrin-carboxylic acids or their salts are described in JP-A 7-76594 and JP-A 7-215895, and can be produced by the methods disclosed in these publications as well as in JP-A 10-210996 and JP-A 10-210996 or analogous methods.

When the physiologically active substance is a pharmaceutical compound, the inventive composition (hereinafter Composition A) can be formulated into an oral formulation such as a tablet (including sugar-coated tablet, film-coated tablet and buccal disintegration tablet), powder, granule, capsule (including soft capsule), liquid and the like; or a parenteral formulation such as an injection, suppository, intravaginal suppository, sublingual tablet, cataplasm, percutaneous formulation by mixing the pharmaceutical compound with pharmaceutically acceptable carriers, surfactants, high molecular compounds and readily soluble cyclodextrin derivatives described above and the like in accordance with a method known per se for producing oral or parenteral compositions, and then can be administered safely. An injection formulation can be used by means of intravenous, intramuscular, subcutaneous or intraorganic administration or by means of direct administration to a lesion. A suppository can be used also by topical administration or rectal administration.

Pharmaceutically acceptable carriers which may be used for producing the inventive Composition A include various organic and inorganic carriers employed routinely as formulation bases, for example, excipients, lubricants, binders and disintegrants for a solid dosage form; and solvents, solubilizing agents, suspending agents, isotonicities, buffering agents, soothing agents for a liquid dosage form. If necessary, conventional additives such as preservatives, antioxidants, colorants, sweeteners, absorbents, wetting agents and the like can also be employed.

Excipients include lactose, white sugar, D-mannitol, starch, cornstarch, crystalline cellulose and light silicic anhydride.

Lubricants include magnesium stearate, calcium stearate, talc and colloidal silica.

Binders include crystalline cellulose, white sugar, D-mannitol, dextrin, hydroxypropyl cellulose, hydroxypropylmethyl cellulose, polyvinyl pyrrolidone, starch, sucrose, gelatin, methyl cellulose and sodium carboxymethyl cellulose.

Disintegrants include starch, carboxymethyl cellulose, calcium carboxymethyl cellulose, sodium croscarmellose, sodium carboxymethyl starch and L-hydroxypropyl cellulose.

Solvents include water for injection, alcohols, propylene glycol, macrogol, sesame oil, corn oil and olive oil.

Solubilizing agents include polyethylene glycol, propylene glycol, D-mannitol, benzyl benzoate, ethanol, trisaminomethane, cholesterol, triethanolamine, sodium carbonate and sodium citrate.

Suspending agents include surfactants such as stearyl triethanolamine, sodium lauryl sulfate, laurylaminopropionic acid, lecithin, benzalkonium chloride, benzethonium chloride, glycerin monostearate and the like; and hydrophilic polymers such as polyvinyl alcohol, polyvinyl pyrrolidone, sodium carboxymethyl cellulose, methyl cellulose, hydroxymethyl cellulose, hydroxyethyl cellulose, hydroxypropyl cellulose and the like.

Isotonicities include glucose, D-sorbitol, sodium chloride, glycerin and D-mannitol.

Buffering agents include buffer solutions of phosphates, acetates, carbonates, citrates and the like.

Soothing agents include benzyl alcohol.

Preservatives include p-hydroxybenzoic esters, chlorobutanol, benzyl alcohol, phenethyl alcohol, dehydroacetic acid and sorbic acid.

Antioxidants include sulfite, ascorbic acid and α-tocopherol.

When the inventive Composition A is formulated into an injection, a pharmaceutical compound is dissolved, suspended or emulsified in an aseptic aqueous or oily fluid.

A carrier for injection may for example be a solvent, solubilizing agent, suspending agent, isotonicity, buffering agent, soothing agent and the like.

The solvent includes water for injection, physiological saline and Ringer's solution. The solubilizing agent includes polyethylene glycol, propylenen glycol, D-mannitol, benzyl benzoate, ethanol, trisaminomethane, cholesterol, triethanolamine, sodium carbonate and sodium citrate. The isotonicity includes glucose, D-sorbitol, sodium chloride, glycerin and D-mannitol. The buffering agent includes buffer solutions of phosphates, acetates, carbonates, citrates and the like. The soothing agent includes benzyl alcohol.

The injection thus obtained may be lyophilized with an aseptic freeze drying machine after, if necessary, removing pyrogens by a method known per se and then stored in the form of powder, or may be stored as it is in a tightly closed container for injection (e.g., ampoule).

While the amount of the pharmaceutical compound in the inventive Composition A may vary depending on the dosage form, it is usually about 0.01 to 99.99% by weight, preferably about 0.1 to 50 % by weight, more preferably about 0.5 to 20% by weight based on the total weight of the composition.

When a surfactant, hydrophilic polymer or readily soluble cyclodextrin derivative is contained in the inventive Composition A, each amount is usually about 1 to 99.99% by weight, preferably about 10 to 90% by weight based on the total weight of the composition, while it may vary depending on the dosage form.

The amount of the pharmaceutically acceptable carrier in the inventive Composition A is usually about 1 to 99.99% by weight, preferably about 10 to 90% by weight based on the total weight of the composition, while it may vary depending on the dosage form.

The inventive Composition A can be administered to mammals (e.g., rat, mouse, guinea pig, monkey, cattle, dog, pig, human and the like) depending on the type of the pharmaceutical compound.

The dose of the inventive Compound A may vary depending on the subject of administration, route of administration and disease. For example, when the Composition A is administered orally as an antihypertensive to an adult (weighing about 60 kg), its dose is about 0.1 to about 20 mg/kg body weight, preferably about 0.2 to about 10 mg/kg body weight, more preferably about 0.5 to about 10 mg/kg body weight of the pharmaceutical compound for antihypertensive, which may be given in one to several portions per day.

When the inventive Composition A is an injection, it can be given by means of intravenous, intramuscular, subcutaneous or intraorganic administration, or can be given directly to a lesion. The dose of an injection may vary depending on the subject of administration, route of administration, disease and the like. For example, when the injection is administered as an antihypertensive to an adult (weighing about 60 kg), its dose is about 0.1 to 500 mg, preferably about 1 to 100 mg, more preferably about 5 to 100 mg of the pharmaceutical compound for antihypertensive per time, which may be given intravenously one to several portions per day.

It is also possible that two or more pharmaceutical compounds are formulated individually and then given to an identical subject simultaneously or sequentially.

When the pharmaceutically active compound is a pesticidal compound, the inventive composition (hereinafter referred to as Composition B) can be formulated into an emulsion, liquid, oil solution, dust, DL (driftless) type dust, granule, microgranule, microgranule F, fine granule F, wettable powder, granular wettable powder, water-soluble powder, flowable formulation, tablet, JUMBO formulation, spray, paste and the like by mixing the pesticidal compound with suitable pesticidal carriers, surfactants, hydrophilic polymers and readily soluble cyclodextrin derivatives described above and the like in accordance with a method known per se for producing pesticidal compositions.

Typically, one or more (preferably one to three) pesticidal compounds are, depending on the purpose of use, dissolved or dispersed in a suitable liquid carrier, or mixed with or adsorbed on a suitable solid carrier, and then mixed if necessary with a surfactant, hydrophilic polymer or readily soluble cyclodextrin derivative. These formulations may be supplemented if necessary with an emulsifier, dispersant, spreading agent, penetrating agent, wetting agent, binder, thickening agent and the like, and can be prepared by a method known per se.

Liquid carriers (solvents) employed include solvents such as water, alcohols (e.g., methanol, ethanol, 1-propanol, 2-propanol, ethylene glycol), ketones (e.g., acetone, methyl ethyl ketone), ethers (e.g., dioxane, tetrahydrofuran, ethylene glycol monomethyl ether, diethylene glycol monomethyl ether, propylene glycol monomethyl ether), aliphatic hydrocarbons (e.g., kerosine, kerosene, fuel oil, machine oil), aromatic hydrocarbons (e.g., benzene, toluene, xylene, solvent naphtha, methylnaphthalene), halogenated hydrocarbons (e.g., dichloromethane, chloroform, carbon tetrachloride), acid amides (e.g., dimethylformamide, dimethylacetoamide), esters (e.g., ethyl acetate, butyl acetate, fatty acid glycerin ester), nitriles (e.g., acetonitrile, propionitrile) and the like. Any of these may be employed alone or in a combination of two or more (preferably one to three) in an appropriate ratio.

Solid carriers (diluents or extenders) employed include vegetable powder (e.g., soybean powder, tobacco powder, wheat powder, wood powder), mineral powder (e.g., clays such as kaolin, bentonite, acid terra alba and clay, talcs such as talc and agalmatolite, silicas such as kieselguhr and mica powder), alumina, sulfur powder, activated carbon, saccharides (e.g., lactose, glucose), inorganic salts (e.g., calcium carbonate, sodium bicarbonate) and hollow glasses (produced by sintering a naturally occurring glass material to encapsulate air bubbles). Any of these may be employed alone or in a combination of two or more (preferably one to three) in an appropriate ratio.

Such a liquid carrier or solid carrier can be employed usually in an amount of about 1 to 99% by weight, preferably about 10 to 99% by weight based on the total weight of the composition.

As an emulsifier, dispersant, spreading agent, penetrating agent, wetting agent and the like, a surfactant is employed as appropriate. Examples of such a surfactant are nonionic surfactants such as polyoxyethylene alkyl ethers (e.g., EMULMIN 110 produced by SANYO KASEI KOGYO), polyoxyethylene alkylaryl ethers (e.g., NONIPOL 85, NONIPOL 100, NONIPOL 160 produced by SANYO KASEI KOGYO), polyoxyethylene lanolin alcohols, polyoyethylene alkylphenol formalin condensates, polyoxyethylene sorbitan fatty acid esters (e.g., Tween 20, Tween 80 produced by KAO, SOLGEN TW-20, SOLGEN TW-80 produced by DAIICHI KOGYO SEIYAKU), polyoxyethylene glyceryl mono-fatty acid esters, polyoxypropylene glycol mono-fatty acid esters, polyoxyethylene sorbitol fatty acid esters, polyoxyethylene castor oil derivatives, polyoxyethylene fatty acid esters, higher fatty acid glycerin esters, sorbitan fatty acid esters, sucrose fatty acid esters, polyoxyethylene polyoxypropylene block polymers, polyoxyethylene fatty acid amides, alkylolamides, polyoxyethylene alkylamine and the like, cationic surfactants such as alkylamine hydrochlorides (e.g., dodecylamine hydrochloride), alkyl quaternary ammonium salts, alkyltrimethyl quaternary ammonium salts (e.g., dodecyltrimethylammonium salts), alkyldimethylbenzylammonium salts, alkylpyridinium salts,alkylisoquinolinium salts, dialkylmorpholinium salts, benzethonium chloride, polyalkylvinylpyridinium salts and the like, anionic surfactants such as higher fatty acid sodium salts (e.g., sodium palmitate), sodium ether carboxylates (e.g., sodium polyoxyethylene lauryl ether carboxylate), amino acid condensates of higher fatty acids (e.g., sodium lauroyl sarcosinate, sodium N-lauroyl glutamate), higher alkyl sulfonates, higher fatty acid ester sulfonates (e.g., lauric acid ester sulfonate), ligninesulfonates (e.g., sodium ligninesulfonate), alkyl sulfosuccinates (e.g., sodium diheptyl sulfosuccinate, sodium dioctyl sulfosuccinate, sodium dinonyl sulfosuccinate), fatty acid amide sulfonates (e.g., oleic acid amide sulfonate), dodecyl benzenesulfonates, diisopropyl naphthalenesulfonates, alkylaryl sulfonate formalin condensates, higher alcohol sulfates (e.g., pentadecan-2-yl sulfate), polyoxyethylene alkyl ether sulfates (e.g., sodium polyoxyethylene dodecyl ether sulfate), polyoxyethylene alkyl phosphates (e.g., dipolyoxyethylene dodecyl ether phosphate), polyoxyethylene alkylaryl phosphates, styrene-maleic acid copolymers, alkyl vinyl ether-maleic acid copolymer and the like, amphoteric surfactants such as N-laurylalanine, N,N,N-trimethylaminopropionic acid, N,N,N-trihydroxyethylaminopropionic acid, N-hexyl-N,N-dimethylaminoacetic acid, 1-(2-carboxyethyl)pyridinium betaine and the like. Any of these may be employed alone or in a combination of two or more (preferably one to five)

Such a surfactant can be used usually in an amount of about 0.1 to 50% by weight, preferably about 0.1 to 25% by weight based on the total weight of the composition.

Binders employed include dextrin (e.g., dextrin ND-S produced by NICHIDEN KAGAKU), sodium salts of carboxymethyl cellulose (e.g., CELLOGEN 5A, 6A, 7A, PR produced by DAIICHI KOGYO SEIYAKU), polycarboxylic acid-based polymeric compounds (e.g., TOXANON GR-30, 31A, GR-50L, GR-60L produced by SANYO KASEI KOGYO; POIS 530, 532A produced by KAO), polyvinyl pyrrolidone, polyvinyl alcohol, sodium ligninesulfonate, calcium ligninesulfonate, sodium polyacrylate, gum arabic, sodium alginate, glucose, sucrose, mannitol and sorbitol. Such binder may be employed usually in an amount of about 0 to 20% by weight based on the total weight of the formulation.

Thickening agents employed include bentonite-based minerals (e.g., highly purified sodium montmorillonite), polyacrylic acids and derivatives thereof, sodium salts of carboxymethyl cellulose (e.g., CELLOGEN 5A, 6A, 7A, PR produced by DAIICHI KOGYO SEIYAKU), white carbons and naturally occurring saccharide derivatives (e.g., xanthane gum, guar gum). Such thickening agents may be employed usually in an amount of about 0.01 to 10% by weight based on the total weight of the formulation.

The content of a pesticidal compound in the inventive Composition B is suitably about 1 to 90% by weight for an emulsion, wettable powder, granular wettable powder, liquid, water-soluble powder, flowable formulation and the like, about 0.01 to 10% by weight for an oil solution, dust, DL dust formulation and the like, and about 0.05 to 10% by weight for a microgranule, microgranule F, fine granule F, granule and the like, while it may vary depending on the purpose of use. An emulsion, wettable powder, granular wettable powder, liquid, water-soluble powder and flowable formulation may be applied after diluting and extending (for example by about 100 to 100,000-fold dilution) with water or the like as appropriate.

When a surfactant, hydrophilic polymer or readily soluble cyclodextrin derivative is contained in the inventive Composition B, the content is usually about 1 to 99.99% by weight, preferably about 10 to 90% by weight based on the total weight of the composition, while it may vary depending on the dosage form.

The content of a pesticidal carrier in the inventive Composition B is usually about 1 to 99.99% by weight, preferably about 10 to 90% by weight based on the total weight of the composition, while it may vary depending on the dosage form.

Application of the inventive Composition B may be conducted in a manner similar to that for applying conventional pesticides, such as aerial application, soil application, foliar application, nursery box application, side row application, seed treatment and the like. For example, a paddy field can be treated by a method known per se (e.g., hand application and mechanical application).

For example, the amount of the inventive Composition B wherein the pesticidal compound is a herbicide, is usually about 0.05 to 50 g, preferably about 0.1 to 10 g of the herbicide per are of a paddy field, and about 0.05 to 50 g, preferably about 0.1 to 10 g of the herbicide per are of a farmland, while it may vary depending on the application site, application season, application mode, subject weeds, crop plant and the like.

When the inventive Composition B is applied to paddy field weeds, it is given preferably by pre-emergence soil treatment or foliage-soil dual treatment.

### Examples

The present invention is further detailed in the following Examples which are not intended to restrict the present invention.

### Example 1

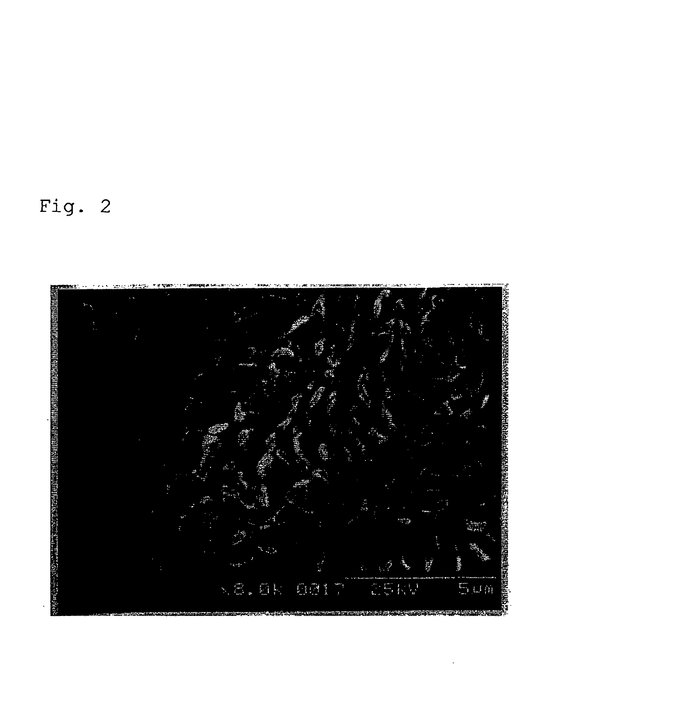

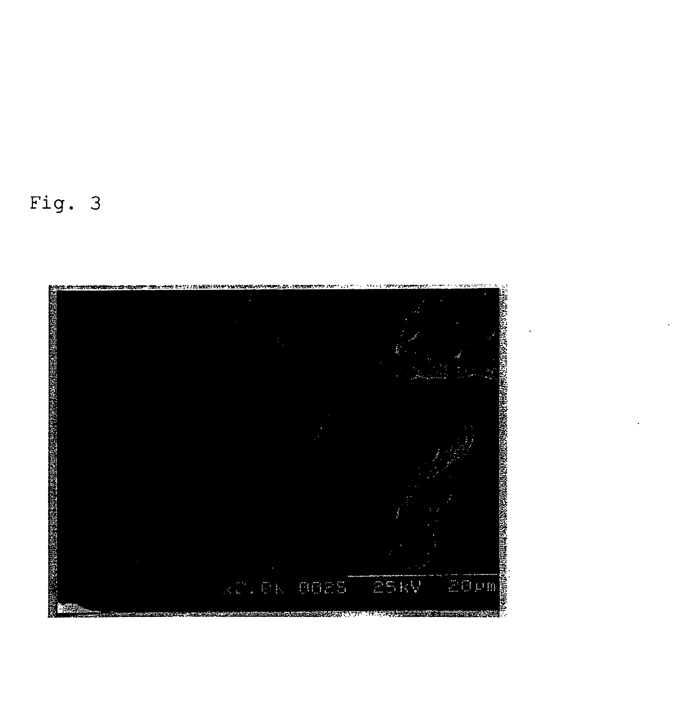

About 1 g of nifedipine was placed in a 90 mL pressure resistance container, which was then warmed to 60°C and received carbon dioxide at the rate of 20 mL/min via a cylinder connected thereto, whereby dispersing the nifedipine in the carbon dioxide. At the time when the pressure in the container reached 20 MPa, the carbon dioxide supply was stopped, and the container was allowed to stand for about 10 minutes, and then depressurized. A SEM photograph of the resultant product is shown in Figure 1, while the magnified photograph is shown in Figure 2. A SEM photograph of nifedipine before the treatment is shown in Figure 3.

### Industrial Applicability

The inventive porous substance has an extremely improved dissolution rate and can readily be handled.

## Claims

1. A physiologically active porous substance obtained by treating a physiologically active solid substance with carbon dioxide in a supercritical or subcritical state or liquid carbon dioxide.

2. A physiologically active porous substance whose weight-average particle size is about 1 µm or more and whose specific surface area is about 1.5 m²/g or more.

3. A physiologically active porous substance according to Claim 2 wherein the weight-average particle size is about 10 µm or more and the specific surface area is about 1.5 m²/g or more.

4. A physiologically active porous substance according to Claim 1 or 2 which is crystalline.

5. A physiologically active porous substance according to Claim 1 or 2 wherein the physiologically active substance is a pharmaceutical compound.

6. A physiologically active porous substance according to Claim 1 or 2 wherein the physiologically active solid substance is a sparingly water-soluble or water-insoluble substance whose solubility in water at 25°C is less than 10 mg/mL.

7. A composition comprising a substance according to Claim 1 or 2.

8. A composition according to Claim 7 comprising a surfactant or a high molecular compound.

9. A composition according to Claim 8 wherein the high molecular compound is a high molecular polymer whose number-average molecular weight is about 3,000 to 30,000.

10. A composition according to Claim 9 wherein the high molecular polymer is one or a copolymer or mixture of two or more selected from the group consisting of (1) poly-fatty acid ester, (2) poly-α-cyanoacrylate, (3) poly-hydroxybutyric acid, (4) polycarbonate selected from polyalkylene oxalate, poly-ortho-ester, poly-ortho-carbonate, polyethylene carbonate and polyethylene propylene carbonate, (5) polyamino acid, (6) polystyrene, (7) polyacrylic acid, (8) polymethacrylic acid, (9) copolymer of acrylic acid and methacrylic acid, (10) silicon polymer, (11) dextran stearate, (12) ethyl cellulose, (13) acetyl cellulose, (14) nitrocellulose, (15) polyurethane, (16) maleic anhydride copolymer, (17) ethylene vinyl acetate copolymer, (18) polyvinyl acetate, (19) polyvinyl alcohol and (20) polyacrylamide.

11. A composition according to Claim 9 wherein the high molecular polymer is polylactic acid, a lactic acid/glycolic acid copolymer, a 2-hydroxybutyric acid/glycolic acid copolymer or a mixture thereof.

12. A composition according to Claim 8 wherein the high molecular compound is a hydrophilic polymer.

13. A composition according to Claim 12 wherein the hydrophilic polymer is one or a mixture of two or more selected from the group consisting of (1) water-soluble polymer selected from hydroxyalkyl cellulose, cellulose derivative, polyalkenyl pyrrolidone, polyalkylene glycol and polyvinyl alcohol; (2) enteric polymer selected from hydroxypropylmethyl cellulose phthalate, hydroxypropylmethyl cellulose acetate succinate, carboxymethylethyl cellulose, cellulose acetate phthalate, methacrylic acid copolymer L and methacrylic acid copolymer S; (3) gastric soluble polymer selected from aminoalkyl methacrylate copolymer E and polyvinyl acetal diethylaminoacetate; (4) carboxymethyl cellulose; (5) Eudragit; (6) carboxyvinyl polymer; (7) polyvinyl alcohol; (8) gum arabic; (9) sodium alginate; (10) alginic acid propylene glycol ester; (11) agar; (12) gelatin and (13) chitosan.

14. A composition according to Claim 7 comprising a readily water-soluble cyclodextrin derivative.

15. A composition according to Claim 14 wherein the readily water-soluble cyclodextrin derivative is a compound represented by Formula: 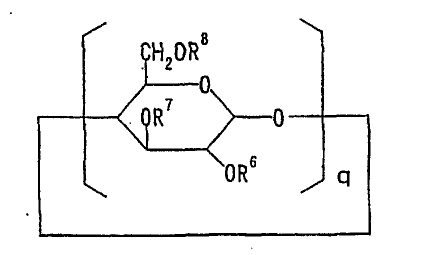 wherein q is an integer of 6 to 12, R⁶, R⁷ and R⁸ are same or different in individual repeating units and each is a dihydroxyalkyl group, sugar residue, hydroxyalkyl group or sulfoalkyl group.

16. A composition according to Claim 15 wherein the dihydroxyalkyl group is a dihydroxy-C₁₋₆ alkyl group, the sugar residue is erythrosyl, threosyl, arabinosyl, ribosyl, glucosyl, galactosyl, glycero-gulco-heptosyl, maltosyl, lactosyl, maltotriosyl or dimaltosyl, the hydroxyalkyl group is a hydroxy-C₁₋₆ alkyl group and the sulfoalkyl group is a sulfo-C₁₋₆ alkyl group.

17. A method for producing a physiologically active porous substance according to Claim 1 or 2 comprising treating a physiologically active solid substance with carbon dioxide in a supercritical or subcritical state or liquid carbon dioxide.

18. A method according to Claim 17 wherein the supercritical or subcritical carbon dioxide or the liquid carbon dioxide is mixed with other solvents.

19. A method according to Claim 17 comprising the steps of (1) placing a physiologically active solid substance in a pressure-resistant container, (2) keeping the temperature of said pressure-resistant container at a level allowing carbon dioxide to be in a supercritical or subcritical state, (3) filling carbon dioxide which may be optionally mixed with other solvents into said pressure-resistant container, (4) stopping filling the carbon dioxide at the time point when the pressure in said pressure-resistant container reaches a level allowing carbon dioxide to be in a supercritical or subcritical state, (5) depressurizing after completing the carbon dioxide treatment and then collecting the resultant physiologically active porous substance.

20. A method according to any one of Claims 17 to 19 wherein the supercritical carbon dioxide is carbon dioxide in a state exceeding both the critical pressure of about 7.38 MPa and the critical temperature of about 304.1 K.

21. A method according to any one of Claims 17 to 19 wherein the subcritical carbon dioxide is carbon dioxide in a state exceeding either the critical pressure of about 7.38 MPa or the critical temperature of about 304.1 K.

22. A method according to Claim 17 comprising the steps of (1) placing a physiologically active substance in a pressure-resistant container, (2) keeping the temperature of said pressure-resistant container at the critical point of carbon dioxide or below, (3) filling carbon dioxide which may be optionally mixed with other solvents into said pressure-resistant container, (4) stopping filling the carbon dioxide under the condition where the pressure in said pressure-resistant container does not exceed the critical point of carbon dioxide, (5) depressurizing after completing the carbon dioxide treatment and then collecting the resultant physiologically active porous substance.

23. A method according to Claim 18 or 19 wherein said other solvents are water, aromatic hydrocarbons, ethers, organochlorine organic solvents, alkylnitriles, nitroalkanes, amides, ketones, fatty acids, alcohols, sulfoxides or mixture solvents thereof.

24. A method according to Claim 18 or 19 wherein said other solvents are water; aromatic hydrocarbons selected from benzene, toluene, ethyl acetate, cyclohexane and xylene; ethers selected from dimethyl ether, diethyl ether, dioxane, diethoxyethane, tetrahydrofuran and 1,2-dimethoxyethane; organochlorine organic solvents selected from dichloromethane, chloroform, carbon tetrachloride and 1,2-dichloroethane; alkylnitriles selected from acetonitrile and propionitrile; nitroalkanes selected from nitromethane and nitroethane; amides selected from N,N-dimethylformamide and N,N-dimethylacetoamide; acetone; fatty acids selected from acetic acid, acetic anhydride and oleic acid; alcohols selected from methanol, ethanol and propanol; dimethyl sulfoxides; or mixture solvents thereof.

25. A method according to Claim 18 or 19 wherein said other solvent is ethanol or acetone.

26. A method according to Claim 18 or 19 wherein the amount of said other solvents is about 1 to 50 % by volume based on the carbon dioxide which is in a supercritical, subcritical or liquid state.
